(19) Europäisches Patentamt / European Patent Office / Office européen des brevets

(11) **EP 4 335 364 A1**

(12) **EUROPEAN PATENT APPLICATION**

(43) Date of publication:
**13.03.2024 Bulletin 2024/11**

(21) Application number: **22205342.3**

(22) Date of filing: **03.11.2022**

(51) International Patent Classification (IPC):
**A61B 5/08** (2006.01)        **A61B 5/00** (2006.01)

(52) Cooperative Patent Classification (CPC):
**A61B 5/0816; A61B 5/7221; A61B 5/7239;**
**A61B 5/725;** A61B 5/0826

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **07.09.2022 PCT/CN2022/117631**

(71) Applicant: **Koninklijke Philips N.V.**
**5656 AG Eindhoven (NL)**

(72) Inventors:
• **ZHANG, Guanqun**
  **Eindhoven (NL)**
• **JIN, Sheng**
  **Eindhoven (NL)**
• **SHI, Jun**
  **5656AG Eindhoven (NL)**
• **YIN, Bin**
  **5656AG Eindhoven (NL)**

(74) Representative: **Philips Intellectual Property &**
**Standards**
**High Tech Campus 52**
**5656 AG Eindhoven (NL)**

(54) **A SYSTEM AND METHOD FOR DETERMINING THE BREATHING RATE OF A SUBJECT**

(57)    A system and method is provided for determining the breathing rate of a subject. A breathing signal is received, and a derivative of a low-pass filtered version of the breathing signal is taken to obtain a derivative signal. In one example, a slope sum filter is applied to transform the derivative signal, and multiple breath intervals are identified from the transformed signal. Breathing characteristics in the filtered breathing signal are obtained using the transformed signal. Suitable breath intervals are selected based on the breathing characteristics and a breathing rate is calculated based on those selected breath intervals.

FIG. 2

EP 4 335 364 A1

**Description**

FIELD OF THE INVENTION

This invention relates to monitoring the breathing rate of a subject, and in particular during sleep.

BACKGROUND OF THE INVENTION

Respiration during sleep is an unconscious behavior regulated by the autonomic nervous system. Sleep apnea is a pathological disorder which results in the cessation of breathing during sleep. Sleep apnea is found to be associated with poor sleep quality, fatigue, drowsiness, stroke, hypertension, heart attack and many other complications. It is estimated that over 1 billion people suffer from sleep apnea worldwide. However, most cases remain unidentified.

Although the diagnosis of sleep apnea is not technically complicated, the procedure is far less applied in clinics than it should be. The limitations of diagnosis in sleep laboratories include expensive medical cost, complicated usage (relying on experienced operators' interpretation), and high intrusiveness for the patient. Therefore, an unobtrusive cost-effective home-based screening solution for sleep apnea would be of significant interest. A solution would be particularly desirable that is feasible for the preliminary identification of sleep disorders for patients at home, without sacrificing the user comfort.

Previous studies proposed numerous different approaches to estimate the breathing rate. The easiest method is to estimate the breathing rate by counting the number of peaks of a filtered breathing waveform within a specified time period. This method requires the fewest computations, and is capable of detecting other inhale/exhale characteristic points, such as when inhale/exhale starts or ends. However, the raw sensor data is often affected by motion artifacts, which compromise the performance of the method.

The breathing rate can also be estimated in the frequency domain, which is typically based on a periodogram. The implementation of a periodogram can be calculated using Fast Fourier Transforms efficiently, with a complexity of the order of $O(N\log N)$, in which N represents the data length for FFT computation. The drawback of the periodogram is its resolution is limited by the data length and FFT length. Thus, precise breathing rate estimation requires longer data, which makes the computation power hungry, which is a particular issue for a battery-based solution. In addition, frequency-based methods are not able to estimate inhalation or exhalation durations since this information is lost when the signal is transformed into the frequency domain.

The ability to estimate the inhalation to exhalation ratio (I/E ratio) (i.e., time domain information), in addition to the breathing rate (i.e., frequency domain information) would be beneficial for clinical needs as an indicator of the user status.

SUMMARY OF THE INVENTION

The invention is defined by the claims.

According to examples in accordance with an aspect of the invention, there is provided a system for determining the breathing rate of a subject, comprising a controller which is configured to:

    receive a breathing signal for the subject;
    low-pass filter the breathing signal to obtain a filtered breathing signal;
    determine a transformed signal at least by obtaining a derivative signal of the filtered breathing signal;
    determine multiple breath intervals from the transformed signal;
    determine breathing characteristics in the filtered breathing signal for the determined multiple breath intervals;
    select breath intervals based on the breathing characteristics; and
    calculate a breathing rate based on the selected breath intervals.

This system analyzes a breathing signal (e.g., from a piezoelectric sensor signal which detects muscle movements or a breathing sensor signal) to calculate a breathing rate. The breathing rate may be used for various purposes, such as for determining sleep apnea conditions from the breathing rate. The system can track the real-time breathing rate, in particular during sleep, efficiently and unobtrusively. The algorithm for determining the breathing rate is computationally simple, for example performed mainly in the temporal domain, which renders the implementation of the device highly affordable. This also enables time domain breathing characteristics to be obtained as well as the basic calculated breathing rate.

The controller may be configured to determine the occurrence and/or severity of sleep apnea based on at least the calculated breathing rate. The breathing rate and its variation over time is thereby used to detect sleep apnea events and/or their severity. For example, during a sleep apnea event, the breathing rate dramatically drops to around zero for a few seconds. If the device detects breathing rate lower than normal values for a certain period, it is a strong indicator of a sleep apnea event. In addition, movements from the chest or abdomen may be detected, caused by the respiratory efforts. In this case, the signal pattern during apneas becomes noticeably different from the normal breathing, which increases the inhalation onset prediction variation.

The controller is for example configured to determine the transformed signal by:

applying a derivative filter to the filtered breathing signal and setting the negative derivative filtered value to zero to obtain the derivative signal; and
applying a slope sum filter to the derivative signal.

The derivative function creates a pulse for each breathing interval. The summing of derivative values using a slope sum filter gives improved signal to noise for the subsequent peak detection (used to identify sequential breath intervals).

The controller is for example configured to:

detect a peak in the transformed signal;
determine a peak position and a valley position in the filtered breathing signal, which correspond to the detected peak in the transformed signal; and
determine the breathing characteristics based on the determined peak position and valley position.

The corresponding peak position and valley position are for example fond by searching back from the time of the peak in the transformed signal to the nearest maximum and minimum of the filtered breathing signal.

For example, a peak and a valley are found in the filtered breathing signal (the timing of these are first examples of breathing characteristics) by searching in the filtered breathing signal for the nearest local maximum and minimum occurring before the peaks of the slope sum filter, SSF, signal.

The controller is for example configured to determine a height difference between an inhalation and exhalation as a magnitude difference between the determined peak position and the neighboring determined valley position in the filtered breathing signal. These are further examples of breathing characteristics. The inhalation/exhalation height difference may thus also be derived from the nearest maximum and minimum of the filtered breathing signal.

The controller is for example configured to determine an inhalation duration and an exhalation duration, based on the determined peak position and valley position in the filtered breathing signal. The controller may then be configured to calculate an inhalation to exhalation ratio based on the determined inhalation duration and the determined exhalation duration.

The controller is for example configured to select multiple breath intervals from the breathing characteristics and derive a candidate breath interval as the average or median of a plurality of most recent selected breath intervals and calculate the breathing rate based on the candidate breath interval.

Thus, suitable breath intervals are identified and selected based on the breathing characteristics (e.g., a suitable height difference between inhalation and exhalation). This facilitates the selection of breath intervals with suitable quality. Furthermore, these suitable breath intervals may be used to calculate the candidate breath interval for deriving the breathing rate.

In one example, the controller is configured to derive a final breath interval by smoothing a plurality of selected breath intervals, and the controller is further configured to calculate the breathing rate based on the final breath interval.

The controller is for example configured to select the breath intervals with:

a height difference within a predetermined height range; and/or
an inhale upstroke which is stably increasing; and/or
a breathing signal or waveform with a mean value within a predetermined mean range; and/or
a breathing signal or waveform with one or more higher order parameters within predetermined respective parameter ranges; and/or
a cosine similarity of consecutive breathing waveforms of the breathing signal within a predetermined similarity range.

In this way, high quality breathing intervals are identified, and they will be those used for determining the breathing rate and optionally also for assessing sleep apnea conditions. The higher order parameters are for example skewness or kurtosis.

The controller is for example configured to derive the final breath interval, FBI, by smoothing the candidate breath intervals.

$$\text{FBI}[n] = \frac{\text{FBI}[n-1]+3\text{BI}[n]}{4}$$

The smoothing is for example performed using: , wherein FBI[n] is the nth final breath interval, FBI[n-1] is the previous (n-1)th final breath interval, and BI[n] is the nth candidate breath interval.

In one embodiment, the most recent calculation from the candidate breath interval is given a weighting of 3 compared to a weighting of 1 for the previously calculated final breath interval. The breathing rate can then be calculated based on 60/FBI[n].

The controller is for example configured to determine inhalation and exhalation durations. The parameters of inhalation and exhalation durations can be used as an indicator of the user respiratory system condition and can enhance sleep apnea determination. For some patients with sleep apnea events or asthma, an inhalation to exhalation ratio may be abnormal.

The controller is for example configured to predict the timing of a start of a next inhalation based on a most recent valley position, a breath interval based on lengths of previously selected breathing intervals and a phase delay caused by the low-pass filtering.

The timing of the start of the next inhalation may for example be predicted using the formula:

$$t\_PO = t\_MIN + BI - IIR\_PO$$

t_PO is the predicted onset time.

t_MIN is the timing of the most recent breath signal minimum (i.e., the timing of the most recent valley position). This breath signal minimum can be detected without needing high signal quality. Thus, the timing of the valley position even from a signal with low quality can be considered as t_MIN.

BI is the breath interval used, such as an average length (or other statistical measure) of the previously selected breathing intervals in a predefined period.

IIR_PO is a phase delay, such as an IIR filter phase offset determined from a function of best fit.

In this way, the system additionally predicts the starting time of a next inhalation for enhancing the sleep apnea determination. A comparison between the predicted timing of the start of the next inhalation and an actual timing of the start of the next inhalation can be conducted. A delay longer than expected (for example, over 0.3 seconds) after a predicted start of a next inhalation of the breath interval may be used as an indicator of a possible sleep apnea event. The occurrence and/or severity of sleep apnea may for example be based on a comparison between the predicted timing of the start of the next inhalation and an actual timing of the start of the next inhalation, and/or the calculated inhalation to exhalation ratio.

By predicting the onset of a next inhalation, the system also has applications in ventilation or paced breathing as well as for detection or analysis of sleep apnea events.

The phase delay, in particular a IIR phase offset, is caused by the filtering operations carried out to the signal (low-pass filtering). For example, the filter may be an infinite impulse response (IIR) filter. By subtracting a phase delay, the distortion created by the filtering (in the particular frequency range for tracking breathing rate during sleep, e.g., 0.1 to 0.6Hz, or 6 breaths per minute to 36 breaths per minute) is compensated so that an accurate estimate of the next inhalation start is obtained.

The next inhalation occurs when the breathing signal has reached it minimum (valley) and is about to increase. Although the minimum (valley) can be detected by comparing values with neighboring data directly, subsequent data samples are needed after the minimum for this purpose. Thus, a time delay is inevitable. Also, the pre-processing (filtering) applied to the raw breathing signal will introduce additional time delay as mentioned above.

The prediction is for example based on an assumption that the breathing intervals should remain almost unchanged within a short duration (for example, around 10 to 20 seconds). Therefore, the next occurrence of inhalation can be predicted given the previous several breathing intervals. In particular, an estimation of the next breathing interval can be taken to be an average of the breathing intervals in the recent predefined period.

The IIR filter, e.g., Butterworth filter, does not have a constant phase delay across the whole frequency. A curve of best fit may be used, such as a 2nd-order polynomial curve, and applied to the phase delay within the valid breathing frequency range (e.g., 0.1 - 0.6 Hz). Using the known current breathing rate, the suitable phase delay can then be subtracted based on the curve fitting. Thereby, the abovementioned additional time delay can be eliminated.

The controller is for example configured to determine inhalation plateaus and/or exhalation plateaus based on height difference, peak location, and valley location.

These plateaus are for example defined as time periods with a breathing signal (the low-pass filtered signal) below a lower threshold and time periods with a breathing signal above a higher threshold.

The controller is for example configured to determine inhalation and exhalation durations, and to determine inhalation and exhalation holding durations. The parameters of inhale and exhale durations can be used as an indicator of the user respiratory system condition. For some patients with asthma, the ratio of inhale-to-exhale duration may be abnormal and is thus of clinical significance for the user.

The system can thus enable efficient in-sleep real-time breathing rate estimation as well as enabling for I/E ratio estimation and inhalation onset prediction. Those different estimation and prediction can be jointly or separately used to determine the occurrence and/or severity of sleep apnea, enhancing the determination accuracy. The computation complexity of the algorithm means the system can be implemented in an embedded system. Usually, the resources such embedded systems, such as ARM Cortex-M4f, are limited in terms of both computation capabilities and memory space.

The invention also provides a breathing monitoring system, comprising:

a sensor arrangement for generating a breathing signal for a subject; and
the system for determining a breathing rate as defined above.

The invention also provides a computer-implemented method for determining a breathing rate of a subject, comprising:

receiving a breathing signal for the subject;
low-pass filtering the breathing signal to obtain a filtered breathing signal;
obtaining a transformed signal at least by obtaining a derivative of the filtered breathing signal;
determining multiple breath intervals from the transformed signal;
determining breathing characteristics in the filtered breathing signal for the determined multiple breath intervals;
selecting breath intervals based on the breathing characteristics; and
calculating a breathing rate based on the selected breath intervals.

The method for example involves calculating the transformed signal by:

applying a derivative filter to the filtered breathing signal and setting the negative derivative filtered value to zero to obtain a derivative signal; and
applying a slope sum filter to the derivative signal.

The method for example obtains breathing characteristics by detecting a peak in the transformed signal, and searching back from the time of the peak in the transformed signal to the nearest maximum and minimum of the filtered breathing signal.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings. In some drawings, A.U. stands for "Arbitrary Units".

Fig. 1 shows a breathing monitoring system;
Fig. 2 shows the processing steps performed by the system of Fig. 1 after receipt of the breathing signal;
Fig. 3 shows a raw breathing signal (top panel), the low-pass filtered signal (bottom panel) and the derivative and SSF of the low-pass filtered signal (middle panel);
Fig. 4 shows a raw breathing signal (top panel), the low-pass filtered signal (middle panel) and the derivative and SSF (bottom panel);
Fig. 5 shows a raw breathing signal (top panel), the low-pass filtered signal (middle panel) and the derivative and SSF (bottom panel); and
Fig. 6 shows a raw breathing signal (top panel), the low-pass filtered signal (middle panel) and the derivative and SSF (bottom panel).

DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a system and method for determining the breathing rate of a subject. A breathing signal is received, and a derivative of a low-pass filtered version of the breathing signal is taken to obtain a derivative signal. In one example, a slope sum filter is applied to transform the derivative signal, and multiple breath intervals are identified from the transformed signal. Breathing characteristics in the filtered breathing signal are obtained using the transformed signal. Suitable breath intervals are selected based on the breathing characteristics and a breathing rate is calculated based on those selected breath intervals.

The invention may be implemented by a smart device that can track the real-time breathing rate during sleep efficiently and unobtrusively. The breathing signal is received from a sensor, for example for measuring the muscle movements caused by respiration. The method can be implemented by a computationally efficient algorithm, which renders the implementation of the device highly affordable. The algorithm performs the analysis mainly in the temporal domain, so that the device can for example derive, in addition to the breathing rate, the time-based inhalation to exhalation ratio from the respiratory signal. This contains information of important clinical significance to the user.

Additionally, in some embodiments, the system can estimate the subsequent oncoming inhalation onset using a mathematical model, which can extend the applicability of the invention to applications which detect when respiration starts, such as ventilation or paced breathing.

The breathing rate is of interest for identifying sleep apnea, and the invention enables an affordable and unobtrusive gateway to identify potential patients with sleep apnea. The early screening of sleep apnea can help reduce morbidity, mortality, and the economic burden for healthcare systems. The invention may also consolidate the need for home ventilation solutions. The inhalation to exhalation ratio and prediction of the inhalation onset can also be used to identify sleep apnea.

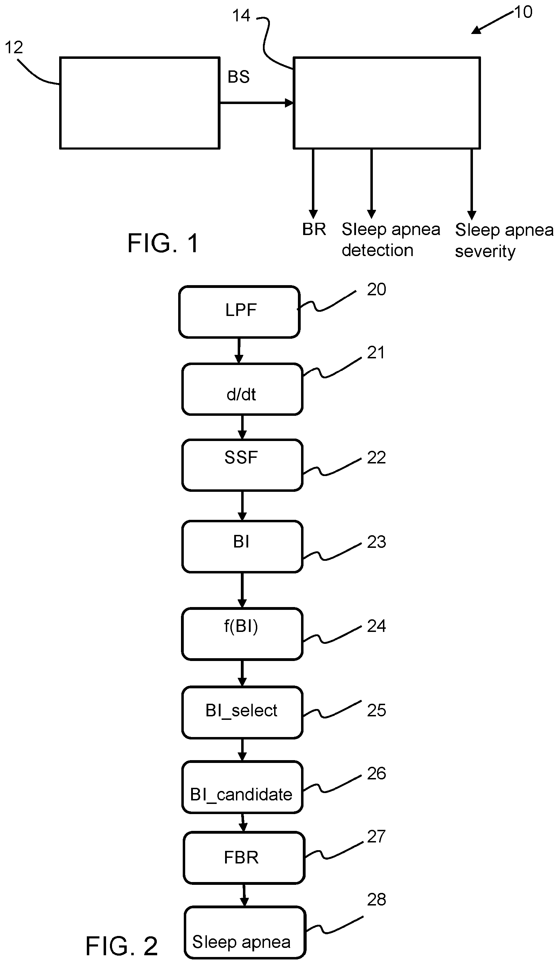

Fig. 1 shows a breathing monitoring system 10, comprising a sensor arrangement 12 for generating a breathing signal BS for a subject and a system 14 for determining a breathing rate BR of the subject. The system 14 comprises a controller which processes the breathing signal BS and derives the breathing rate, and optionally also provides detection of sleep apnea events and determines a severity of detected sleep apnea events.

Fig. 2 shows the processing steps after receipt of the breathing signal. The breathing signal can be obtained by any suitable sensor forming part of a sleep monitoring application for detecting respiratory vibrations or flows, such as a pillow or a mattress, or a bed side system using a radar or ultrasound, or piezoelectric sensor, or an air flow sensor. The raw sensor data is transmitted to the controller for further processing.

In step 20, the breathing signal is low-pass filtered. This is a preprocessing stage. Previous studies often apply low-pass or band-pass filters to the raw sensor signal for attenuating unwanted frequency components. The lower bound of the low-pass or band-pass filter is for example set as 0.05 or 0.1 Hz, and the upper bound of the filter is often set at 0.5 Hz or 1 Hz. For example, a low-pass second order Butterworth filter may be used remove the high frequency noise, with the cut-off frequency at 0.5 Hz.

Note that if a band-pass filter is applied, this includes a low-pass filter function. Thus, the definition of a "low-pass filter" is intended to include low-pass and band-pass filtering.

In steps 21 and 22, the low-pass filtered signal is transformed, by obtaining a derivative signal in step 21 using a derivative filter and by obtaining a transformed, SSF (slope sum filter), signal by applying a SSF filter to the derivative signal.

The raw sensor signal may have a wandering baseline and subtle perturbations, which is challenging for the algorithm to detect true peaks caused by breathing. The transformation takes advantage of the waveform characteristics.

A derivative filter is first applied to the low-pass filtered signal. Negative values of the derivatives are set to zero:

$$y[t]=MAX(0, (x[t+window]-x[t-window])/(2*window))$$

$t$ is the current time of the signal $x[t]$. $y[t]$ is the derivative signal at the current time, whereas $x[t+window]$ and $x[t-window]$ are the values of window duration after and before $y[t]$, respectively. The window width is selected to achieve the optimal performance, for example 0.4 seconds. The function of MAX() returns the larger value of the two parameters. Since the first parameter is zero, y[t] will always be non-negative.

The slope sum filter (SSF) is applied to the derivatives. The SSF function sums all values within a fixed width moving window (for example, 0.8 seconds). Since all derivatives are non-negative, values calculated from SSF represent the increasing momentum of the filtered signal. This is because the SSF function adds up recent non-negative values derived from the derivative filter within the most recent 0.8 seconds, for example. Therefore, the more increasing the derivative signal is increasing, the larger value of the SSF calculation.

The SSF values are for example calculated as:

$$SSF[t]=y[t-window]+y[t-window+1]+\cdots+y[t+window-1]+y[t+window]$$

The transformation (derivative and SSF) has the advantage of improved signal-to-noise ratio for peak detection, so that the trivial perturbations are attenuated.

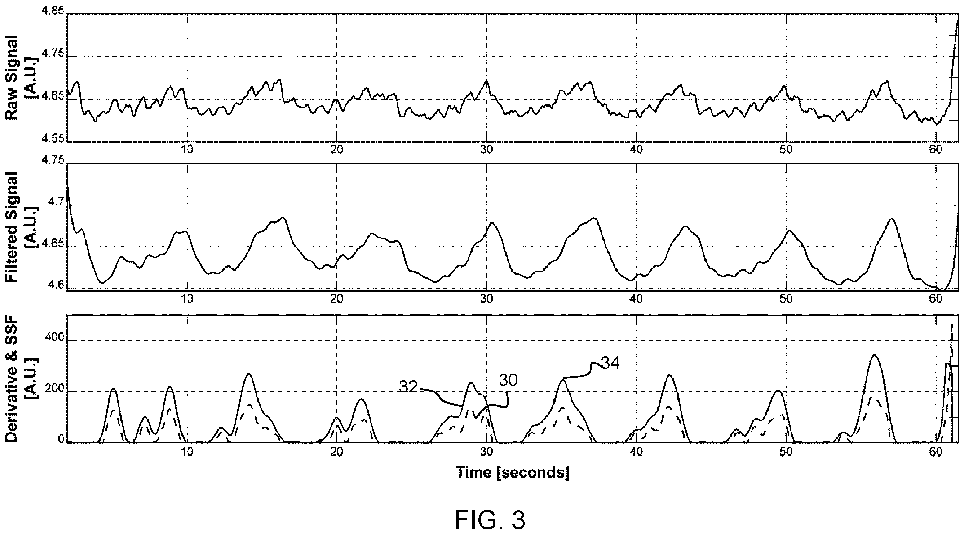

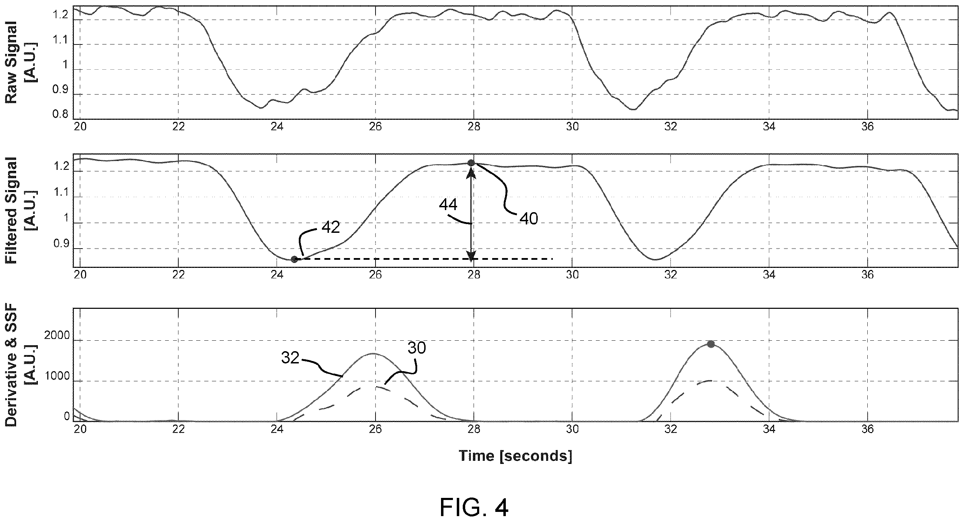

Fig. 3 shows a raw breathing signal (top panel), the low-pass filtered signal (middle panel) a scaled derivative of the low-pass filtered signal (bottom panel, plot 30) and the SSF signal (bottom panel, plot 32). The value of the SSF signal 32 is much larger than the original derivative signal 30. The difference is much larger than represented, in that the derivative signal is multiplied by a constant (20 in this example) so that the scaled derivative signal can be seen, together with the SSF signal. Similarly, in the bottom panels of Figs 4 to 6, the derivative signal has also been multiplied by a constant to be shown together with the SSF signal.

Even though the raw breathing signal is smoothed by the low-pass filtering, as the middle panel depicts, it still has trivial perturbations which may confuse the peak detection algorithm. After the filtered signals are transformed by the derivative and SSF functions, minor perturbations are significantly attenuated so that the peak detection becomes much easier. A detected peak is shown in the middle panel as point 34.

In step 23, breath intervals BI are identified from the SSF signal.

The breath intervals are determined by fiducial point detection. In particular, the breath interval is measured from the SSF waveform (plot 32), while other inhalation/exhalation characteristics (e.g., when does inhalation/exhalation start/end or the inhale/exhale hold duration) are estimated from the filtered breath signal (middle panel).

One SSF peak will correspond to one breathing interval. The algorithm finds the local maximum of the SSF peak in the searching window mentioned above, i.e. from SSF[t-window] to SSF[t+window]. SSF[t] is the current value of the SSF signal, and the window is for example of a pre-defined duration such as 0.4 seconds. Therefore, minor peaks around a higher peak will not be recognized as a separate SSF peak.

The SSF peaks 34 are found from the SSF waveform as mentioned above. The SSF peak is defined as a local maximum exceeding a threshold in a moving window. For different users, the threshold can adapt automatically to match the personalized signal magnitude. Otherwise, the signal caused by the subtle movement artifact of an obese person may have the comparable magnitude with that generated by a normal breathing of another slim person.

Once there is a newly detected SSF peak 34, a peak and a valley are found from the filtered signal (bottom panel of Fig. 3) by searching back for the nearest local maximum and minimum occurring before the SSF peak.

This process is show in Fig. 4. The top panel shows the raw breathing signal, and the middle panel shows the low-pass filtered signal. The bottom panel shows the derivative signal as plot 30 and the SSF signal as plot 32.

The bottom panel of Fig. 4 shows that a peak position for the SSF signal is found at around 33s, for example. This is determined using the searching window as discussed above, as the local maximum position in the searching window.

The search then extends back in time for a peak and valley in the filtered signal. As shown in the middle panel, point 40 is determined as a as peak, and point 42 is determined as a valley.

Breath characteristics f(BI) for those breath intervals are obtained from the original low-pass filtered signal, in the time domain in step 24.

For example, as shown in Fig. 4, the height difference between an inhalation and exhalation is shown as 44. It is the magnitude difference between peaks and neighboring valleys. The peak and the neighboring valley preferably belong to one breath interval.

Other breath characteristics may also be obtained.

For example, the inhale plateau threshold is determined as a portion (e.g.,7.5%) of the height difference between an inhalation and exhalation and the exhale plateau threshold is determined as a portion (e.g.,7.5%) of the height difference between an inhalation and exhalation. The inhale plateaus start where the signal value is equal to the peak amplitude minus the threshold (i.e., 7.5% of height difference between an inhalation and exhalation) and the location is before the peak. The exhale plateaus start (inhale ends) where the signal value is equal to the peak amplitude minus the threshold (i.e., 7.5% of height difference between an inhalation and exhalation) and the location is after the peak. Similarly, the exhale plateaus start where the signal value is equal to the valley amplitude plus the threshold and the location is before the valley. The inhale plateaus start (exhale ends) where the signal value is equal to the valley amplitude plus the threshold and the location is after the valley.

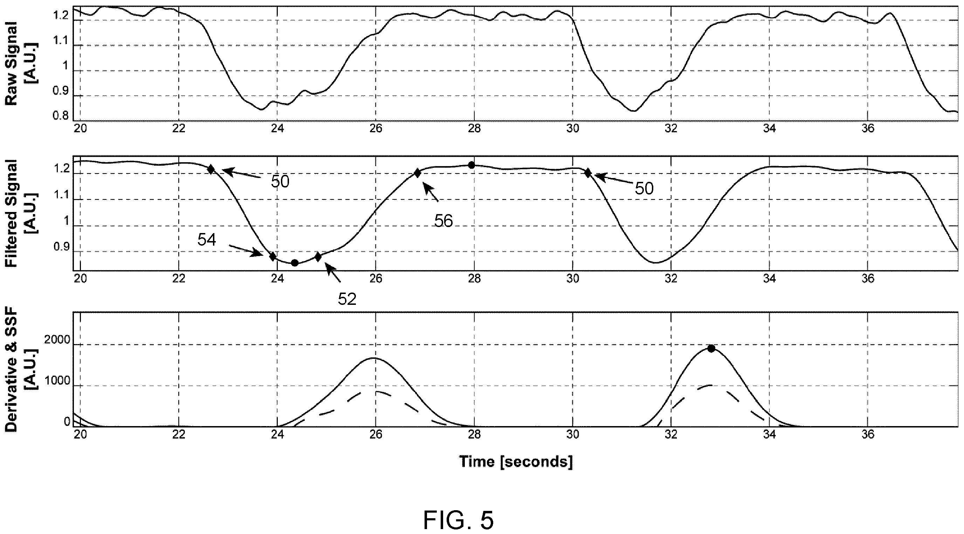

Fig. 5 shows the exhale start time as point 50 and the inhale start time as point 52. The exhale plateau is shown as the signal between points 54 and 52, whereas the inhale plateau is shown as the signal between points 56 and 50.

The inhalation duration is defined as the difference between one inhale starting time and a subsequent exhale starting time. The inhale holding duration is defined as the difference between one inhale plateau starting time and a subsequent exhale starting time. Similarly, the exhalation duration is defined as the difference between one exhale starting time and a subsequent inhale starting time. The exhale holding duration is defined as the difference between one exhale plateau starting time and a subsequent inhale starting time.

In summary, the inhale plateau and the exhale plateau are determined based on the peak and valley position, and height difference between an inhalation and exhalation and threshold, which are derived from the peak and valley positions. Furthermore, the exhale duration, the inhale duration, the exhale holding duration, and the inhale holding duration are derived based on the exhale plateau and the inhale plateau. Preferably, if the signal quality is not sufficient for a specific breathing interval, the inhalation duration, exhale starting time, the exhale holding duration, and inhale starting time will not be calculated at all.

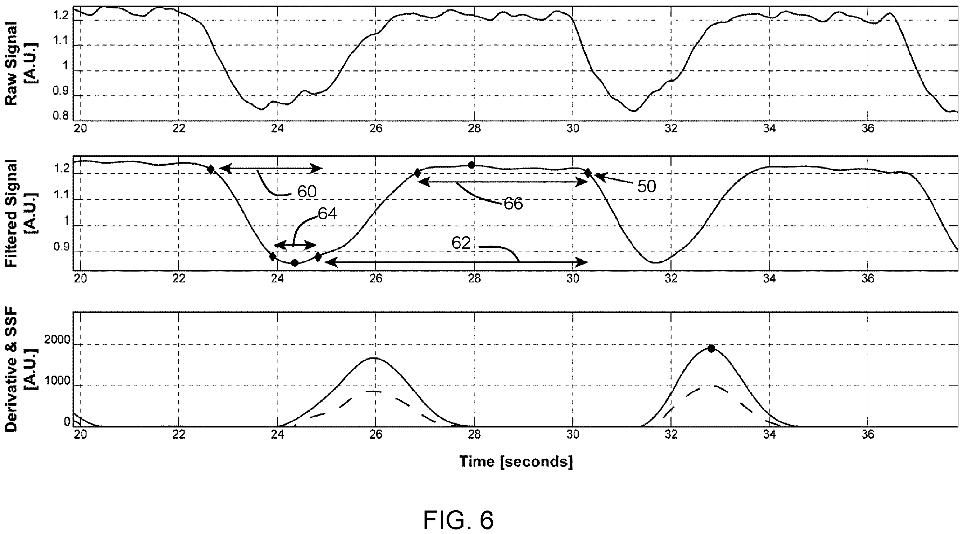

Fig. 6 shows the exhale duration as time period 60 and the inhale duration as time period 62. The exhale holding time is shown as 64 and the inhale holding time is shown as 66.

Thus, there are various (time domain) breathing characteristics that may be determined, and they may be used in various ways to assess when a breath interval is a suitable choice for determining the breathing rate. Additionally, the breathing characteristics can be used to evaluate the predicted timing of the start of the next inhalation and/or the inhalation to exhalation ratio.

Thus, based on the breathing characteristics, suitable breath intervals are selected in step 25 which may be used for the subsequent determination of the breathing rate. The suitable breath intervals are those breath intervals with suitable quality. In this way, to guarantee the calculation accuracy, the signal of each breath interval is screened to check the signal quality.

By way of example, two rules may be used to check for waveform abnormalities:

(i) Check if the height difference between an inhalation and exhalation is too small or too large;
(ii) Check that the inhale upstroke is not increasing stably (in other words, the inhale upstroke without a monotonic increase signifies an irregular breathing pattern).

Other breathing characteristics which may be considered are:

(iii) a breathing waveform with a mean value within a predetermined mean range;
(iv) a breathing waveform with one or more higher order parameters (e.g., skewness or kurtosis) within predetermined respective parameter ranges;
(v) a cosine similarity of consecutive breathing waveforms within a predetermined similarity range.

Those rules can be considered jointly or separately. Breath intervals will be marked as of suitable quality if the set rules for compliance are passed (e.g., the first two rules above). Otherwise, the quality of the breath interval is marked as poor.

From those suitable breath intervals, a candidate breath interval is calculated in step 26 in Fig. 2. This is an artificial breath interval obtained by combining the selected suitable breath intervals, for example the average or median of a set of the most recent suitable breath intervals.

The number of breath intervals involved has to exceed a minimum required threshold within a pre-defined time. For example, at least 5 breaths should exist within the recent 90 seconds. It no sufficient numbers of breaths are available, the calculation will generate a message indicating no breath can be found. The breath interval value has units of seconds by dividing a signal sample index by the sampling frequency. In other words:

$$\text{Breath interval (seconds)} = \text{signal sample index (data points)} / \text{sampling frequency (data points/second)}.$$

A final breath interval, and hence breathing rate, is obtained in step 27 based on a smoothing function. In an example, the final breath interval is based on smoothing the current candidate breath interval with the previous final breath interval:

$$FBI[n] = (FBI[n-1] + 3BI[n])/4$$

n is the indexing number of breath intervals. BI[n] is the latest candidate breath interval. FBI[n] is the final breathing interval, which is converted to a final breathing rate as 60 (seconds) divided by the final breath interval. The units are the number of breaths per minute:

$$Breathing\ rate[n] = 60/FBI[n]$$

In step 28, sleep apnea events and optionally their severity may be evaluated based on the calculated breathing rate. For example, during a sleep apnea event, the breathing rate dramatically drops to around zero for a few seconds. Thus, by detecting when there is a breathing rate lower than normal values for a certain period, a sleep apnea event may be detected.

Other characteristics of the breathing signal may also be used as indicators of a sleep apnea event, or to detect the severity of a sleep apnea event. For example, a respiratory effort signal may also be used.

A known measure of severity of a sleep apnea event is the apnea-hypopnea index. It may also be combined with a measure of oxygen desaturation (using a pulse oximeter) to derive a sleep apnea severity score. A count of apnea events and hypopnea events may be performed in known manner.

Step 28 may also include other post-processing functions. The breathing rate is for example processed for visualization on a display screen, for storage in memory or for transfer to a remote device such as a smartphone via Bluetooth or Wi-Fi. The post-processing can perform customized strategies, such as alerting the user or other staff if the breathing rate has been out of the normal range for over a certain period.

The processing described above is used to determine the breathing rate and optionally other breathing characteristics, in particular time-domain characteristics of the breathing signal. Examples of the time-domain characteristics include the inhalation/exhalation ratio, where the inhalation duration is the elapsed time from one inhalation starting point to its subsequent exhalation starting point, and the exhalation duration is the remaining elapsed time when the inhalation duration is excluded from one breathing cycle. The ratio can be estimated as the average inhalation duration divided by the average exhalation duration within the same time windows as the breathing rate.

A further option is to predict the timing of the next breathing interval, i.e. the next onset of inhalation. This makes use of the estimation of the breath interval that has already been made based on the most recent breath cycles. In addition, the prediction takes account of the phase delay of the filters used to process the raw data. The phase delay of an IIR filter is for example a parabolic function over the frequency range of interest, such as 0.1 - 0.6 Hz in this case. By way of example, the IIR phase delay can be estimated using a regressed a second order polynomial function with group phase delay, which estimates the IIR phase delay from the breathing rate determined from recent breathing intervals (BI).

The oncoming inhale onset time is predicted as:

$$t\_PO = t\_MIN + BI - IIR\_PO$$

t_PO is the predicted onset time.

t_MIN is the timing of the most recent breath signal minimum.

BI is an average length of the previous selected breathing intervals (i.e., breathing interval with a suitable quality) in a predefined period.

IIR_PO is the IIR filter phase offset determined from the polynomial function of best fit.

The IIR filter phase offset is for compensating for the low-pass filter, since the inhale/exhale features are obtained from the filtered signal (not the derivative or SSF signal).

An example will be explained with reference to Fig. 4.

The purpose of the processing is to predict where the next inhalation will be, in particular the point in time when the breathing signal has reached it minimum (valley) and is about to increase. This is point 42 in Fig. 4.

The minimum can be detected by comparing with neighboring data directly, but this needs subsequent data samples after the minimum for confirmation giving a time delay, hence making a prediction impossible. The pre-processing applied to the raw breathing signal also introduces additional time delay, namely the IIR phase delay discussed above.

The prediction is based on the assumption that breathing interval durations remain almost unchanged within a short duration (for example, around 10 to 20 seconds).

For example, in Fig. 4, the inhalation valley has been detected at around 24.2 seconds. Note that this is the detected timing within the filtered signal, not the true timing, as a result of the time delay caused by the filtering.

However, the true timing is desired, for example for controlling other devices.

For a prediction of this 31.8 seconds timing point, t_MIN is 24.2 seconds. Assuming the most recent breath interval calculation (based on a set of recent selected breath intervals as discussed above) is 7.5 seconds, this would predict the next breathing valley to be at around 24.2 + 7.5 = 31.7 seconds.

The filter delay is visible in Fig. 4, as the time shift between the top and middle panels. The fitting of a curve to fit the phase delay within the valid breathing frequency (0.1 to 0.6 Hz, or 6 breaths to 36 breaths per minute) enables the filter delay to be estimated. The phase delay is for example 0.3 seconds and is then subtracted from the signal valley time (31.7 seconds) accordingly. The next valley of the raw signal is thus predicted to occur at 31.7-0.3 = 31.4 seconds, which aligns with the timing for the top panel of Fig. 4.

In principle, the actual inhale onset should be quite close to the predicted value under normal breathing conditions. When the sleep apnea occurs, the pattern of respiration has changed. Therefore, the predicted inhale onset might be much farther away from the actual occurrence, for example, over 0.3 seconds.

The invention provides a method for determining the breathing rate of a subject by analyzing a breathing signal for the subject. The method is preferably implemented in software which controls a processor to perform the signal analysis. The software comprises a computer program, i.e. computer program code means, which is run by a processor forming part of the analysis system. The computer program is for example embodied as a computer program medium (i.e. a memory device).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A system for determining a breathing rate of a subject, comprising a controller (14) which is configured to:
   receive a breathing signal (BS) for the subject;

   (20) low-pass filter the breathing signal to obtain a filtered breathing signal;
   (21, 22) determine a transformed signal at least by obtaining a derivative signal of the filtered breathing signal;
   (23) determine multiple breath intervals from the transformed signal;
   (24) determine breathing characteristics in the filtered breathing signal for the determined multiple breath intervals;
   (25) select breath intervals based on the breathing characteristics; and
   (27) calculate a breathing rate based on the selected breath intervals.

2. The system of claim 1, wherein the controller is further configured to:
   (28) determine the occurrence and/or severity of sleep apnea based on the calculated breathing rate.

3. The system of claim 1 or 2, wherein the controller is configured to determine the transformed signal by:

   applying a derivative filter to the filtered breathing signal and setting the negative derivative filtered value to zero to obtain the derivative signal; and
   applying a slope sum filter to the derivative signal.

4. The system of any one of claims 1 to 3, wherein the controller is configured to:

detect a peak in the transformed signal;
determine a peak position and a valley position in the filtered breathing signal, which correspond to the detected peak in the transformed signal; and
determine the breathing characteristics based on the determined peak position and valley position.

5. The system of claim 4, wherein the controller is configured to determine a height difference between an inhalation and exhalation as a magnitude difference between the determined peak position and the neighboring determined valley position in the filtered breathing signal.

6. The system of claim 4 or 5, wherein the controller is configured to determine an inhalation duration and an exhalation duration, based on the determined peak position and valley position in the filtered breathing signal.

7. The system of claim 6, wherein the controller is configured to calculate an inhalation to exhalation ratio based on the determined inhalation duration and the determined exhalation duration.

8. The system of any one of claims 1 to 7, wherein the controller is configured to derive a candidate breath interval as the average or median of a plurality of most recent selected breath intervals, and the controller is further configured to calculate the breathing rate based on the candidate breath interval.

9. The system of claim 8, wherein the controller is configured to derive a final breath interval by smoothing a plurality of selected breath intervals, and the controller is further configured to calculate the breathing rate based on the final breath interval.

10. The system any one of claims 1 to 9, wherein the controller is configured to select the multiple breath intervals with one or more of the following breathing characteristics:

a height difference within a predetermined height range;
an inhale upstroke which is increasing stably;
a breathing signal with a mean value within a predetermined mean range;
a breathing signal with one or more higher order parameters within predetermined respective parameter ranges; and/or
a cosine similarity of consecutive breathing waveforms of the breathing signal within a predetermined similarity range.

11. The system of claim 10, wherein the controller is configured to predict the timing of a start of a next inhalation based on a most recent valley position, a breath interval based on lengths of previously selected breathing intervals and a phase delay caused by the low-pass filtering.

12. The system of claim 11, wherein the controller is configured to determine the occurrence and/or severity of sleep apnea based on a comparison between the predicted timing of the start of the next inhalation and an actual timing of the start of the next inhalation, and/or the calculated inhalation to exhalation ratio.

13. A breathing monitoring system, comprising:

a sensor arrangement for generating a breathing signal for a subject; and
the system for determining a breathing rate of any one of claims 1 to 12.

14. A computer-implemented method for determining a breathing rate of a subject, comprising:
receiving a breathing signal for the subject;

(20) low-pass filtering the breathing signal to obtain a filtered breathing signal;
(21, 22) determining a transformed signal at least by obtaining a derivative signal of the filtered breathing signal;
(23) determining multiple breath intervals from the transformed signal;
(24) determining breathing characteristics in the filtered breathing signal for the determined multiple breath intervals;
(25) selecting breath intervals based on the breathing characteristics; and
(27) calculating a breathing rate based on the selected breath intervals.

15. A computer program comprising computer program code means which is adapted, when said program is run on a computer, to implement the method of claim 14.

12

14

BS

10

BR   Sleep apnea   Sleep apnea
     detection      severity

## FIG. 1

LPF — 20

d/dt — 21

SSF — 22

BI — 23

f(BI) — 24

BI_select — 25

BI_candidate — 26

FBR — 27

Sleep apnea — 28

## FIG. 2

FIG. 3

FIG. 4

FIG. 5

FIG. 6

Europäisches
Patentamt

European
Patent Office

Office européen
des brevets

# EUROPEAN SEARCH REPORT

**Application Number**

**EP 22 20 5342**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/107268 A1 (ABOA LEGIS OY [FI]) 23 July 2015 (2015-07-23) | 1,2,4-15 | INV. A61B5/08 A61B5/00 |
| A | * page 6, lines 12-29 * <br> * page 8, lines 23-31; figures 1A-1B * <br> * the whole document * | 3 | |
| X | US 2020/397308 A1 (SARKAR SHANTANU [US] ET AL) 24 December 2020 (2020-12-24) | 1,2,4-15 | |
| A | * paragraphs [0031], [0060], [0097] – [0099] * <br> * the whole document * <br> * paragraphs [0142], [0148] * | 3 | |
| A | CN 111 803 041 A (ZHEJIANG SHUYUAN INTELLIGENT TECH CO LTD) 23 October 2020 (2020-10-23) * claim 5 * | 3 | |

| | |
|---|---|
| | **TECHNICAL FIELDS SEARCHED (IPC)** |
| | **A61B** |

The present search report has been drawn up for all claims

| Place of search | Date of completion of the search | Examiner |
|---|---|---|
| The Hague | 10 August 2023 | Furlan, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 5342

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2023

| Patent document cited in search report | | Publication date | Patent family member(s) | | Publication date |
|---|---|---|---|---|---|
| WO 2015107268 | A1 | 23-07-2015 | EP 3094248 | A1 | 23-11-2016 |
| | | | JP 6310086 | B2 | 11-04-2018 |
| | | | JP 2017502782 | A | 26-01-2017 |
| | | | WO 2015107268 | A1 | 23-07-2015 |
| US 2020397308 | A1 | 24-12-2020 | AU 2020308341 | A1 | 16-12-2021 |
| | | | CN 113950285 | A | 18-01-2022 |
| | | | DE 202020005685 | U1 | 18-01-2022 |
| | | | EP 3986270 | A1 | 27-04-2022 |
| | | | US 2020397308 | A1 | 24-12-2020 |
| | | | WO 2020263568 | A1 | 30-12-2020 |
| CN 111803041 | A | 23-10-2020 | NONE | | |

EPO FORM P0459

For more details about this annex : see Official Journal of the European Patent Office, No. 12/82